Europäisches Patentamt

European Patent Office

Office européen des brevets

(19)

(11) Numéro de publication : **0 683 153 A1**

## (12) DEMANDE DE BREVET EUROPEEN

(21) Numéro de dépôt : 95401038.5

(22) Date de dépôt : 04.05.95

(51) Int. Cl.[6] : **C07C 53/08,** C07C 51/215,
B01J 27/195

(30) Priorité : 20.05.94 FR 9406168

(43) Date de publication de la demande :
22.11.95 Bulletin 95/47

(84) Etats contractants désignés :
BE DE FR GB IT NL

(71) Demandeur : RHONE-POULENC CHIMIE
25, Quai Paul Doumer
F-92408 Courbevoie Cédex (FR)

(72) Inventeur : Bordes, Elisabeth
26, rue Léon Bouchard
F-95470 Vemars (FR)
Inventeur : Tessier, Laurent
119, rue des Pyrénées
F-75020 Paris (FR)
Inventeur : Gubelmann-Bonneau, Michel
22, rue Raynouard
F-75016 Paris (FR)

(74) Mandataire : Dubruc, Philippe et al
RHONE-POULENC CHIMIE,
Direction de la Propriété Industrielle,
25, Quai Paul Doumer
F-92408 Courbevoie Cédex (FR)

(54) **Catalyseurs à base d'hétéropolyacide et leur utilisation pour la préparation d'acides carboxyliques aliphatiques.**

(57) La présente invention a trait à un procédé de préparation d'acide carboxyliques aliphatiques par oxydation ménagée des alcanes correspondants, avec une source d'oxygène, en présence d'un catalyseur dont la phase active est obtenu à partir d'un hétéropolyacide de formule (I)

$$[A_aB_b]_f[C_cD_dE_eO_x]^{f-}$$

dans laquelle :
— A représente au moins un cation monovalent choisi parmi l'hydrogène, un métal alcalin, ou l'ion ammonium ;
— B représente $VO^{2+}$, $VO^{3+}$, un ion d'un métal alcalino-terreux ou d'un métal des colonnes VII A, VIII, I B, IV B et V B de la classification périodique des éléments ;
— C représente W et/ou Mo ;
— D représente le phosphore, l'arsenic, l'antimoine, le silicium, le germanium et/ou le bore ;
— E représente un élément choisi parmi le vanadium éventuellement en combinaison avec au moins un métal des colonnes V A, VII A et VIII ou le chrome ;
— $f = a + \alpha b$ ; avec $\alpha$ dépendant de la charge de l'ion B, soit égal à 2, 3 ou 4 ;
— c varie entre 5 et 20-e inclus ;
— d varie entre 1 et 5 inclus ;
— e varie entre 1 et 9 inclus.
L'invention a de même pour objet une composition obtenue à partir de dioxyde de titane en tant que support et d'un hétéropolyacide de formule (I) précitée.
Elle a enfin pour objet un catalyseur dont la phase active est la composition définie ci-dessus.

EP 0 683 153 A1

La présente invention a trait à un procédé de préparation d'acides carboxyliques par oxydation ménagée des alcanes correspondants, en présence d'un catalyseur dont la phase active est obtenue à partir d'un hétéropolyacide.

La présente invention concerne par ailleurs une composition obtenue à partir d'un hétéropolyacide, et de dioxyde de titane en tant que support, ainsi qu'un catalyseur comprenant ladite composition en tant que phase active.

Par convention, on utilisera dans ce texte le terme hétéropolyacide (ou HPA) pour désigner à la fois les hétéropolyacides et les polyoxométallates.

L'obtention d'acides carboxyliques, tel que notamment l'acide acétique, par oxydation ménagée de l'alcane, tel que l'éthane, est un procédé en plein développement car il présente l'avantage d'utiliser une matière première dont le coût est très concurrentiel par rapport à celles employées dans les technologies existantes.

Parmi les procédés connus de ce type, on peut citer ceux mettant en jeu, outre l'éthane et l'oxygène, des catalyseurs à base, au moins, d'oxydes de vanadium, de phosphore et de rhénium. Les sélectivités en acide acétique sont de l'ordre de 30 % mais elles restent cependant inférieures à celles de l'éthylène coproduit. En outre, ce procédé nécessite d'introduire dans le réacteur de fortes quantités d'eau et d'un gaz inerte comme l'hélium ou l'azote.

Un autre procédé consiste à faire réagir l'éthane et l'oxygène en présence de catalyseurs à base d'au moins un mélange d'oxydes de molybdène, de vanadium, de niobium et d'antimoine, ou encore à base d'oxydes de molybdène, vanadium, rhénium, antimoine, niobium et calcium. La sélectivité en acide acétique par mise en oeuvre de tels catalyseurs est améliorée par rapport à celle obtenue selon le procédé précédent. Toutefois les conditions de réactions sont exigeantes. En effet, il est nécessaire d'effectuer l'oxydation dans des conditions combinant une température élevée, c'est-à-dire de l'ordre de 300 à 400°C, et une pression importante, de l'ordre de 20 à 30 bar. Par ailleurs, les procédés de préparations de telles formules catalytiques ne sont pas simples à mettre en oeuvre.

Il est par ailleurs connu d'utiliser des catalyseurs dont la phase active comprend des oxydes de vanadium, ce dernier étant au degré d'oxydation (IV), de titane et/ou de phosphore. Dans le cas de ces catalyseurs, les performances sont intéressantes en termes de sélectivité en acide acétique. Cependant, le flux d'alimentation comprend plus de 90 % de gaz diluant, ce qui a pour résultat une productivité très faible.

Ainsi que l'on peut le constater, on ne dispose pas, à l'heure actuelle, de procédé de préparation d'acide carboxylique aliphatique, et plus particulièrement l'acide acétique, par oxydation d'un alcane pouvant être développé à l'échelle industrielle et mettant en jeu des catalyseurs dont la fabrication est simple.

Or il a été trouvé de façon totalement inattendue que l'utilisation de catalyseurs dont la phase active a été obtenue à partir d'un hétéropolyacide de formule (I), qui sera définie ci-après, dans la réaction précitée, permettait de pallier les inconvénients mentionnés ci-dessus.

Ainsi, le procédé selon l'invention combine une sélectivité augmentée en acide carboxylique formé, dans des conditions de mise en oeuvre peu contraignantes permettant d'envisager une exploitation de la réaction à l'échelle industrielle.

De plus, le procédé selon l'invention peut être mis en oeuvre avec une quantité élevée d'alcane. Ceci représente un avantage certain par rapport aux procédés employant des mélanges réactionnels comprenant de grandes quantités de gaz diluant inerte. En effet, l'alcane, qui n'est converti qu'en partie, peut être recyclé très facilement dans la réaction.

En outre, le procédé selon l'invention est mis en oeuvre à une température plus basse que celles habituellement employées. Or il est tout à fait surprenant de pouvoir activer les alcanes, et tout particulièrement l'éthane, à des températures pouvant être aussi faibles que 150°C - 250°C, connaissant la réactivité plus faible de l'éthane par rapport à des hydrocarbures saturés à plus longue chaîne ou encore celle des hydrocarbures saturés par rapport aux hydrocarbures insaturés, aromatiques ou non.

Ainsi, le procédé selon l'invention consiste à préparer un acide carboxylique aliphatique par oxydation ménagée de l'alcane correspondant, avec une source d'oxygène, en présence d'un catalyseur dont la phase active est obtenue à partir d'un hétéropolyacide de formule (I)

$[A_a B_b]_f[C_c D_d E_e O_x]^{f-}$ dans laquelle :

- A représente au moins un cation monovalent choisi parmi l'hydrogène, un métal alcalin, ou l'ion ammonium ;
- B représente $VO^{2+}$, $VO^{3+}$, un ion d'un métal alcalino-terreux ou d'un métal des colonnes VII A, VIII, I B, IV B et V B de la classification périodique des éléments ;
- C représente W et/ou Mo ;
- D représente le phosphore, l'arsenic, l'antimoine, le silicium, le germanium ou le bore ;
- E représente un élément choisi parmi le vanadium et au moins l'un des métaux des colonnes V A, VII A et VIII ou le chrome ;

- f = a + αb ; avec α dépendant de la charge de l'ion B, soit égal à 2, 3 ou 4 ;
- c varie entre 5 et 20-e inclus ;
- d varie entre 1 et 5 inclus ;
- e varie entre 1 et 9 inclus.

Ici et pour toute la description, les mentions relatives au tableau de la classification périodique des éléments font référence à celui paru dans le supplément au bulletin de la Société Chimique de France (n°1 - janvier 1966).

L'invention a de même pour objet une composition obtenue à partir de dioxyde de titane en tant que support et d'un hétéropolyacide de formule (I) $[A_aB_b]_f[C_cD_dE_eO_x]^{f-}$, dans laquelle A, B, C, D, E, a, b, c, d, e et f ont la même signification que celle donnée précédemment.

Un autre objet de l'invention est enfin constitué par un catalyseur comprenant la composition précitée en tant que phase active.

Dans ce qui va suivre, les termes dioxyde de titane et oxyde de titane ont la même signification.

Mais d'autres avantages et caractéristiques de l'invention apparaîtront plus clairement à la lecture de la description qui va suivre.

Ainsi, le procédé selon l'invention consiste à mettre en oeuvre un catalyseur dont la phase active est obtenue à partir d'un hétéropolyacide de formule (I) précitée.

Plus particulièrement, l'élément B est choisi parmi les ions $VO^{2+}$, $VO^{3+}$, $Cu^{2+}$, $Fe^{3+}$, $Co^{2+}$, $Ag^+$, $Ni^{2+}$, $Mn^{2+}$, $Mg^{2+}$, $Bi^{3+}$, $Sn^{2+}$, $Sn^{4+}$.

De préférence, l'élément B est choisi parmi les ions $VO^{2+}$, $VO^{3+}$.

En ce qui concerne l'élément E différent du vanadium, celui-ci est plus particulièrement choisi parmi le chrome, le manganèse, le fer, le cobalt, le nickel.

Selon une variante préférée de l'invention, l'hétéropolyacide entrant dans la préparation de la phase active correspond à la formule (I) dans laquelle D est le phosphore, E le vanadium, d vaut 1, C est compris entre 1 et 3 et la somme de c +e est égale à 12.

Selon un mode de réalisation particulier, le catalyseur employé présente une phase active comprenant, en outre, un support.

A titre de support convenable à la réalisation de ce mode particulier, on peut citer par exemple, les dioxydes de titane, silicium, zirconium, cérium, étain, l'alumine, la silice-alumine, ces composés pouvant être utilisés seuls ou en mélange.

De préférence, le support est choisi parmi les dioxydes de titane ou de zirconium, le premier étant préféré.

Selon une variante de ce mode de réalisation, la phase active du catalyseur présente un rapport atomique, (C + E) / élément métallique du support, compris entre 0,1 et 30 %. Par élément métallique du support, on entend le titane, le zirconium, le cérium, etc.

De préférence, le rapport atomique précité est compris entre 5 et 20 %.

Dans le cas où la phase active comprend un support, la répartition entre les deux constituants de la phase active est telle que l'hétéropolyacide de formule (I) est plus particulièrement dispersé à la surface dudit support.

Selon un premier mode de réalisation, le catalyseur est employé sous forme massique, c'est-à-dire un catalyseur ne comprenant que la phase active obtenue à partir de l'hétéropolyacide et le cas échéant du support.

Selon un second mode de réalisation, le catalyseur est employé sous forme diluée, c'est-à-dire que la phase active précitée est mélangée à un inerte.

Dans ce dernier cas, la phase active peut être soit déposée sur celui-ci, soit enrobée ou encore y être mélangée.

A titre de matériaux susceptibles d'être utilisés comme inertes, on peut citer : l'argile frittée, la magnésie, le silicate de magnésium, la terre de diatomée. Ces types de matériaux inertes peuvent être utilisés sous forme poreuse ou non. De préférence, l'inerte utilisé l'est sous forme non poreuse. Si nécessaire, on peut effectuer un émaillage de celui-ci afin de le rendre tel.

Les matériaux céramiques, du type de la cordiérite, la mullite, la porcelaine, les nitrures de silicium et de bore, le carbure de silicium, peuvent aussi être utilisés comme inerte.

Le catalyseur mis en oeuvre dans le procédé selon l'invention, dilué ou non, se présente sous forme de particules ou de monolithe.

Si le catalyseur est constitué de particules, la granulométrie de celles-ci dépend du mode d'utilisation du catalyseur. Elle peut donc varier dans de larges limites, comme notamment être comprise entre quelques micromètres et une dizaine de millimètres. Plus particulièrement, et à titre indicatif, un catalyseur utilisé en lit fixe, présente une répartition granulométrique généralement comprise entre 0,5 et 6 mm. La granulométrie des particules d'un catalyseur utilisé en lit fluidisé ou mobile, est habituellement comprise entre 5 et 700 microns, et de préférence comprise entre 5 et 200 microns pour 80 % des particules.

Si le catalyseur est constitué de particules, toutes les formes conviennent à la mise en oeuvre de l'invention. Ainsi, le catalyseur peut se trouver par exemple sous la forme de billes ou d'anneaux. Par anneaux on désigne des objets creux dont la section est circulaire, parallélépipèdique, ellipsoïdale entre autres. On peut de même envisager tout autre type de structure complexe obtenu par extrusion de l'inerte par exemple (croix, étoile, etc.).

D'une façon classique, la quantité d'inerte entrant dans la composition du catalyseur varie dans de larges limites, dépendant, la plupart du temps, du mode de mise en forme du catalyseur.

Ainsi, les catalyseurs obtenus par enrobage ou dépôt de la phase active sur le matériau inerte présentent une quantité de phase active variant habituellement entre 0,1 et 30 % et de préférence entre 2 et 20 % en poids total de catalyseur (phase active + inerte).

Dans les cas où le catalyseur comprend la phase active dispersée dans un inerte, la quantité de phase active est comprise entre 1 et 90 % en poids total de catalyseur.

Selon un mode de réalisation préféré de l'invention, la phase active du catalyseur enrobe l'inerte présent.

Le catalyseur peut être préparé selon toute méthode classique, simple et reproductible, ce qui constitue un avantage supplémentaire de l'invention.

Les hétéropolyacides sont des composés connus et l'homme du métier pourra se référer aux publications les concernant pour les préparer.

En ce qui concerne plus particulièrement les hétéropolyacides, c'est-à-dire les composés pour lesquels A représente un atome d'hydrogène, on peut notamment mettre en oeuvre deux types de procédés.

Selon une première méthode, convenant plus particulièrement à la préparation d'hétéropolyacides dans lesquels b vaut 0 et d vaut 1, on porte à reflux, dans l'eau pendant 24 heures, un mélange comprenant les éléments constitutifs de l'hétéropolyacide, de préférence sous forme d'oxydes.

Une autre méthode d'obtention des hétéropolyacides du même type que précédemment et pour lesquels la valeur de c est comprise entre 6 et 12, consiste à préparer une solution des éléments constitutifs de l'HPA, présents sous la forme de sels de métaux alcalins ou alcalino-terreux. Celle-ci est obtenue en solubilisant lesdits composés dans de l'eau.

Une fois la solution obtenue, celle-ci est neutralisée par l'ajout d'un acide minéral tel que l'acide chlorhydrique notamment. Le produit résultant est extrait du milieu avec de l'éther puis mis en contact avec de l'eau distillée pour obtenir une solution aqueuse, de laquelle on peut cristalliser l'hétéropolyacide.

A l'issue de chacune de ces deux méthodes, il est possible d'obtenir par évaporation ou cristallisation, l'hétéropolyacide désiré. Celui-ci peut être utilisé directement en tant que catalyseur dans la réaction selon l'invention, éventuellement après avoir subi une étape de calcination qui sera décrite ci-après.

D'une façon usuelle et dans le cas où le catalyseur mis en oeuvre comprend un support, la mise en contact de l'hétéropolyacide avec ledit support est effectuée par le moyen, par exemple, d'une imprégnation à sec, bien que d'autres voies ne soient pas exclues a priori. A ce titre, on peut citer le mélange des divers constituants, hétéropolyacide et support sous forme solide par exemple.

Selon la méthode d'imprégnation, on met en contact le support tel que défini auparavant, avec une solution d'hétéropolyacide dans une quantité telle que le rapport atomique, (C + E) / élément métallique du support, soit compris entre 0,1 et 30 % et de préférence entre 5 et 20 %.

On sèche ensuite la suspension résultante. Cette étape de séchage peut avantageusement être effectuée en deux temps : le premier consistant à évaporer le solvant ou dispersant du mélange, plus particulièrement l'eau, jusqu'à siccité, le second à sécher la pâte ainsi obtenue.

Généralement, la première étape est réalisée à une température variant de 20 à 100°C sous vide ou non, pendant la durée nécessaire pour obtenir une pâte.

L'évaporation est habituellement effectuée sous agitation.

La pâte obtenue est ensuite séchée, dans un second temps, sous une atmosphère de préférence non réductrice, comme l'oxygène ou l'air par exemple, pendant une durée moyenne de 15 heures.

La température de séchage est habituellement comprise entre 100 et 150°C.

Il est à noter que d'autre méthodes de séchage peuvent être envisagées, telles que par exemple, le séchage de la suspension par atomisation dans tout type d'appareillage et dans des conditions connus de l'homme du métier.

Le produit séché est ensuite soumis à une étape de calcination.

Celle-ci est réalisée, de façon classique, sous une atmosphère non réductrice. Avantageusement on utilise l'air, mais l'oxygène pourrait tout aussi bien être employé.

La température de calcination est habituellement comprise entre 200 et 500°C.

Généralement, la durée de l'opération varie entre 1 et 24 heures.

Préalablement et/ou postérieurement à l'étape de calcination, le produit séché peut subir une étape de désagglomération.

Dans le cas où le catalyseur mis en oeuvre dans le procédé selon l'invention, comprend un inerte, on met de préférence en oeuvre la méthode d'enrobage.

Ainsi, on met en contact l'inerte, de préférence sous forme de particules rugueuses, et la phase active dans un mélangeur à fort cisaillement (appareils type LODIGE) ou dans un appareil de granulation (drageoirs, sous forme de tambour ou assiette).

L'opération est effectuée en général à une température variant entre 20 et 150°C pendant la durée nécessaire à l'enrobage du matériau inerte par la quantité désirée de phase active, plus particulièrement sous air, pendant au moins 30 minutes.

Les particules ainsi obtenues sont habituellement calcinées à une température comprise entre 300 et 500°C.

La durée de la calcination est généralement d'au moins 3 heures.

Bien entendu, tous ces modes de préparation de l'hétéropolyacide, de la mise en contact dudit HPA avec le support et/ou l'inerte, ne sont donnés qu'à titre indicatif et ne peuvent en aucun cas constituer une liste exhaustive.

Ainsi que cela a été mentionné auparavant, le procédé selon l'invention consiste à effectuer une oxydation ménagée de l'alcane avec une source d'oxygène, en présence d'un catalyseur tel que décrit précédemment.

Il n'y a pas de conditions particulières concernant la qualité de l'alcane mis en oeuvre. Cependant, pour des questions évidentes de séparation de l'acide formé, on préfère utiliser de l'éthane présentant une pureté d'au moins 90 %.

La réaction d'oxydation ménagée de l'alcane est mise en oeuvre en présence d'une source d'oxygène. Celle-ci peut être à base d'oxygène pur ou dilué dans un gaz inerte ou encore de protoxyde d'azote.

On peut donc effectuer, de façon avantageuse, la réaction d'oxydation en utilisant de l'air comme source d'oxygène.

Selon un mode de réalisation particulier de la présente invention, le rapport molaire entre l'alcane et l'oxygène est inférieur à 20. Plus particulièrement, ce rapport est compris entre 0,01 et 0,2 ou entre 0,6 et 18.

Selon un mode préféré de l'invention, ledit rapport est compris entre 0,6 et 18.

La réaction d'oxydation peut en outre être effectuée en présence d'un diluant.

Par diluant, on entend l'eau ou les gaz inertes comme l'azote ou les gaz rares, tels que l'hélium ou l'argon par exemple. Il est à noter que les gaz diluants peuvent aussi être des gaz recyclés de la réaction comme les oxydes de carbone. Le diluant peut être une combinaison de tout ou partie des gaz précités.

La composition du mélange gazeux, soit l'alcane, la source d'oxygène, le cas échéant le gaz diluant peut varier dans de larges limites.

Sauf mention contraire, tous les pourcentages indiqués par la suite, sont exprimés en pourcent molaire du mélange gazeux.

D'une façon générale, la teneur en alcane dans le mélange gazeux, est comprise entre 0,1 et 99,9%.

Selon un mode particulier de réalisation de l'invention, la composition du mélange gazeux est telle qu'elle se trouve en dehors de la zone d'explosivité dudit mélange.

Plus particulièrement, et afin d'avoir un mélange gazeux dont la composition se trouve commodément en dehors du domaine d'explosivité, ladite teneur en alcane est comprise entre 0,1 et 3 % ou entre 10 et 99 %.

Préférentiellement, la teneur en alcane dans le mélange gazeux précité est comprise entre 10 et 99 %.

La teneur en oxygène dans le mélange gazeux mis en oeuvre varie de même dans une large gamme de concentration. Elle est en effet comprise entre 0,1 et 99,9 %.

Selon un mode de réalisation plus particulier, la teneur en oxygène dans le mélange gazeux varie entre 1 et 90 % ou entre 97 et 99,9%.

De préférence, l'oxygène contenu dans ledit mélange est comprise entre 1 et 90%.

Selon une variante particulière de l'invention, la réaction est mise en oeuvre en présence d'eau. Dans ce cas, la teneur en eau dans le mélange gazeux est plus particulièrement comprise entre 0 et 70 %.

Selon un mode de réalisation particulier, la teneur en eau dans le mélange précité est de 0 à 20%.

La teneur en gaz inerte dans le mélange, l'azote étant préféré, varie habituellement entre 0 et 70%.

Plus particulièrement le mélange comprend entre 0 et 20 % de gaz diluant.

Le mélange gazeux est donc mis au contact du catalyseur selon l'invention.

Il est à noter que la mise en contact du mélange gazeux avec le catalyseur peut avoir lieu en effectuant au préalable un pré-mélange de tous les constituants dudit mélange, ou bien encore en les introduisant de façon séparée. On peut de même envisager une injection étagée de l'alcane, ou de préférence, de la source d'oxygène, dans le réacteur.

Les deux dernières méthodes mentionnées peuvent être de préférence retenues dans le cas d'exploitations industrielles, pour des raisons de sécurité du procédé. En effet, de cette façon, on évite d'avoir à évacuer une quantité trop importante de calories du fait de l'exothermie de la réaction, et l'on diminue les risques de se trouver dans la zone d'explosivité du mélange.

Le dispositif dans lequel le procédé selon l'invention est mis en oeuvre fait partie des dispositifs classiques pour les réactions catalytiques en phase gaz ; ces derniers pouvant être utilisés en continu ou en discontinu.

Ainsi, on peut effectuer la réaction dans un réacteur dans lequel le catalyseur est en lit fixe, fluidisé, transporté, en lits fixes alternés ou encore dans un réacteur à inversion de flux. De préférence, le procédé selon l'invention est mis en oeuvre dans des réacteurs à lit fixe, fluidisé ou encore transporté. Pour plus de précisions à ce sujet, on pourra se référer à la publication "Réacteurs Chimiques" de P. Trambouze, parue dans les Techniques de l'ingénieur (J 4020,12-1993), ainsi qu'à la publication "Selective oxidation in riser reactor" de R.M. Contractor et A.W. Sleight, parue dans Catalysis Today (vol. 3, (1988), 175-184), concernant plus particulièrement les réacteurs à lit transporté.

La température de réaction est en général située entre 100 et 400°C, de préférence entre 150 et 350°C.

La pression totale du mélange gazeux réactionnel varie en général entre 0,1 et 20 bar absolus.

Le débit gazeux est fixé de telle sorte que le temps de contact, calculé dans les conditions normales de température et pression, soit compris entre 0,1 et 30 secondes.

De préférence, le temps de contact est compris entre 0,5 et 20 secondes. Il est rappelé que le temps de contact correspond au rapport entre le volume du réacteur et le débit total des gaz.

L'acide carboxylique formé est séparé des sous-produits ou des réactifs, classiquement par refroidissement et condensation d'un mélange acide/eau.

Les composés restés sous forme gazeuse, plus particulièrement l'alcane et les oxydes de carbone, peuvent, d'une façon totalement avantageuse, être recyclés au réacteur. Recycler l'alcane présente un avantage tout à fait évident pour l'homme du métier ; avantage qui est de diminuer au maximum les pertes en ce réactif. Il est cependant à noter que le recyclage de l'alcane et des oxydes de carbone présente un avantage supplémentaire, ayant trait à l'absorption des calories dégagées pendant la réaction d'oxydation. En effet, ces gaz ont des capacités calorifiques non négligeables les rendant particulièrement indiqués dans cet objectif, et par conséquent, le fait de la recycler permet de minimiser les risques d'augmentation locale de la température (points chauds).

En cas de recyclage de ces gaz, et plus spécialement en ce qui concerne les oxydes de carbone, il est tout à fait clair pour l'homme du métier qu'il est nécessaire de purger régulièrement ces gaz qui risquent de s'accumuler, au fur et à mesure des recyclages.

La composition selon l'invention va maintenant être décrite.

Celle-ci est obtenue à partir de dioxyde de titane en tant que support et d'un hétéropolyacide de formule (I) telle que décrite précédemment.

Selon une variante préférée de l'invention, la formule de l'hétéropolyacide correspond à la formule (I) dans laquelle D est le phosphore, E le vanadium, c est compris entre 1 et 3 inclus, d vaut 1, et la somme de c + e est égale à 12.

L'oxyde de titane présent dans le catalyseur mis en oeuvre dans le procédé de l'invention peut être indifféremment sous forme de $TiO_2$ anatase, rutile, brookite ou encore bronze (symbolisé (B)), ou leurs mélanges.

De préférence, la forme allotropique de l'oxyde de titane est choisie parmi les formes anatase, rutile, ou leurs mélanges.

L'oxyde de titane entrant dans la composition de la phase active présente en outre une surface spécifique, mesurée selon la méthode B.E.T., comprise entre 1 et 150 m²/g. Plus particulièrement la surface spécifique est comprise entre 10 et 120 m²/g.

Plus particulièrement la composition selon l'invention est telle que le rapport atomique (C + E) / Ti est compris entre 0,1 et 30 % et de préférence compris entre 5 et 20 %.

L'invention a par ailleurs pour objet un catalyseur dont la phase active est la composition décrite précédemment.

Selon un mode de réalisation particulier, le catalyseur selon l'invention comprend un inerte, outre la phase active obtenue à partir d'oxyde de titane et d'hétéropolyacide de formule (I).

Une première variante est constituée par un catalyseur de type enrobé. Dans ce cas, la quantité de composition selon l'invention est comprise entre 0,1 à 30 % en poids, par rapport au poids total de catalyseur. De préférence ladite teneur est comprise entre 2 et 20 % en poids.

Une seconde variante est constituée par un catalyseur dont la phase active est dispersée dans l'inerte. Selon cette variante la quantité de composition est comprise entre 1 à 90 % en poids, par rapport au poids total du catalyseur.

Tout ce qui a été dit auparavant concernant l'hétéropolyacide, l'inerte, les proportions des divers éléments les uns par rapport aux autres, ainsi que les modes de préparation du catalyseur, reste valable pour cette partie et ne sera donc pas repris ici.

Des exemples concrets mais non limitatifs de l'invention vont maintenant être présentés.

## EXEMPLES

Dans ce qui va suivre, les performances des catalyseurs sont calculées de la manière suivante :
La conversion est calculée à partir de la somme des rendements en produits observés.

- conversion de l'éthane (% mol)

$$\text{conv. éthane} = \frac{\Sigma \text{ nbre de moles de produits X observés}}{(\text{nbre moles éthane entré})} \times 100$$

- sélectivité d'un produit X (acide acétique, éthylène, produits de combustion) (% mol)

$$\text{sél. X} = \frac{\text{nbre de moles de produit X}}{\Sigma \text{ nbre de moles de produits X observés}} \times 100$$

## EXEMPLE 1

Synthèse d'un hétéropolyacide de formule $H_4PMo_{11}VO_{40}/TiO_2$(catalyseur 1) :

On mélange 8,23 g de $MoO_3$, 0,52 g de $V_2O_5$ et 0,66 g de $H_3PO_4$ 85 % dans 150 ml d'eau distillée. Après 24 heures de reflux, on ajoute 30 g de $TiO_2$ anatase (DT 51, Rhône-Poulenc - 86 m²/g) à la solution rouge pourpre et on évapore à sec sous vide.

Le produit est calciné à 320°C sous air pendant 4 heures.

On obtient un catalyseur dont le rapport (Mo + V) / Ti est de 16,7 %.

L'analyse par Résonance Magnétique Nucléaire à l'angle magique du $^{31}P$ (unité déplacement en ppm par rapport à une référence externe de $H_3PO_4$ en solution aqueuse à 85 %). Cette analyse permet de confirmer que la structure de l'HPA est au moins partiellement conservée.

Cette technique donne des signaux entre -3 et -4 ppm (singulet) pour l'HPA massique en faible intéraction avec le support $TiO_2$ ; à environ +2 ppm (singulet) pour l'HPA en forte intéraction avec le support $TiO_2$ ; entre -10 et -15 (signal large mal résolu) pour l'HPA décomposé. Il est à noter que la présence d'un signal entre -3 et -4 ppm après la synthèse du catalyseur est préférable pour obtenir de bonnes performances catalytiques.

## EXEMPLE 2

Synthèse d'un hétéropolyacide de formule $H_5PMo_{10}V_2O_{40}$(catalyseur 2) :

Les sels de sodium des différents constituants sont utilisés dans les proportions suivantes :
- 1 mole de $Na_2MoO_4$, $2H_2O$ (242,0 g),
- 0,4 mole de $NaVO_3$ (48,8 g),
- 0,1 mol de $Na_2HPO_4$ (14,1 g).
a) $NaVO_3$ est dissous dans 500 ml d'eau bouillante. On ajoute $Na_2HPO_4$ et on laisse refroidir.
b) $Na_2MoO_4,2H_2O$ est mis en solution dans 500 ml d'eau à froid.
c) La solution a) est acidifiée par HCl 37 % jusqu'à obtenir une coloration rouge pourpre, et on y ajoute rapidement la solution b), puis 400 ml d'HCl 37 % au goutte-à-goutte en refroidissant dans un bain de glace.
d) L'éthérate de l'acide $H_5PMo_{10}V_2O_{40}$ est recueilli par extraction de la solution c) à l'éther.
3 phases dans l'ampoule à décanter:

bas: éthérate de l'acide,
milieu: phase aqueuse orangée,
haut: éther.

L'éthérate est hydrolysé par la moitié de son volume en eau et l'ensemble est laissé à cristalliser à 4°C.
Les cristaux de $H_5PMo_{10}V_2O_{40},nH_2O$ obtenus sont calcinés à 320°C sous air.

## EXEMPLE 3

Synthèse d'un hétéropolyacide de formule $H_5PMo_{10}V_2O_{40}/TiO_2$(catalyseur 3) :

9,08g de l'hétéropolyacide obtenu à l'exemple 2 sont mis en solution dans 150 ml d'eau avec 14g de $H_2O_2$ à 50 %.

On ajoute alors 30 g de $TiO_2$ sous forme anatase (DT 51, Rhône-Poulenc - 86 m²/g) et l'on évapore le mélange à sec sous vide.

Le produit est séché pendant 16 heures à 140°C, puis calciné sous air à 320°C pendant 4 heures.

On obtient un catalyseur dont le rapport (Mo + V) / T est de 16,7 %.

## EXEMPLE 4

Synthèse d'un hétéropolyacide de formule $H_6PMo_9V_3O_{40}$(catalyseur 4) :

On utilise le même mode opératoire que celui décrit dans l'exemple 2 avec les proportions suivantes :
- 0,45 mole de $Na_2MoO_4,2H_2O$ (108,90 g),
- 0,6 mole de $NaVO_3$ (73,20 g),
- 0,1 mole de $Na_2HPO_4$ (14,2 g).
On obtient un catalyseur dont le rapport (Mo + V) / Ti est de 16,7 %.

## EXEMPLE 5 Comparatif

Synthèse d'un hétéropolyacide de formule $H_3PMo_{12}O_{40}/TiO_2$ ;(catalyseur 5)

On procède comme décrit dans l'exemple 3 en utilisant $H_3PMo_{12}O_{40},nH_2O$ du commerce (Fluka).
Le produit est calciné à 320°C sous air pendant 4 heures.
On obtient un catalyseur dont le rapport Mo/Ti est de 16,7 %.

## EXEMPLE 6

Cet exemple illustre les performances des catalyseurs décrits précédemment dans la réaction d'oxydation ménagée de l'éthane en acide acétique.

On introduit 3 g de catalyseur en poudre dans un réacteur continu en INOX et à lit fixe, équipé d'un chauffage par bain de sable fluidisé et de deux chromatographes en ligne, l'un fonctionnant avec un détecteur à ionisation de flamme et l'autre avec un détecteur thermoconductimétrique.

Les gaz d'alimentation sont contrôlés par des débitmètres massiques.

Le flux d'alimentation (exprimé en mol %) est constitué de:

éthane / $O_2$ / $N_2$=85 / 5 / 10.

La vitesse volumique horaire est de 3200 $h^{-1}$.

La pression du réacteur est maintenue constante à 1 ou 11 bar absolus.

La température est de 250 °C.

| Catalyseur | P (bar absolus) | conv.éthane (%) | sél. AcOH (%) | sél. $C_2H_4$(%) | sél. $CO_x$(%) |
|---|---|---|---|---|---|
| 1 | 1 | 0,5 | 69 | 21 | 10 |
| 2 | 11 | 0,5 | 65 | 30 | 5 |
| 3 | 1 | 0,6 | 61 | 26 | 13 |
| 4 | 11 | 0,4 | 78 | 17 | 5 |
| 5 | 1 | 0,1 | 50 | 39 | 11 |

Il est rappelé que la conversion théorique de l'éthane en acide acétique est de 3,9%.

## EXEMPLE 7

On procède comme pour l'exemple 6, excepté le fait que le flux d'alimentation (exprimé en mole %) est constitué de:

éthane / $O_2$ / $H_2O$ / $N_2$=62 / 17 / 10 / 11

La vitesse volumique horaire est de 2400 $h^{-1}$.

La pression du réacteur est maintenue à 6 bar absolus.

La température est de 275°C.

Dans ces conditions, la convension de l'éthane est de 7,7 %.

La sélectivité en acide acétique est de 70 %, la sélectivité en éthylène de 10 %, la sélectivité en COx de 20 %.

**Revendications**

1. Procédé de préparation d'acides carboxyliques aliphatiques par oxydation ménagée des alcanes correspondants avec une source d'oxygène, caractérisé en ce que l'on effectue la réaction en présence d'un catalyseur dont la phase active est obtenue à partir d'un hétéropolyacide de formule (I) : $[A_aB_b]_f[C_cD_dE_eO_x]^{f-}$ dans laquelle :
   - A représente au moins un cation monovalent choisi parmi l'hydrogène, un métal alcalin, ou l'ion ammonium ;
   - B représente $VO^{2+}$, $VO^{3+}$, un ion d'un métal alcalino-terreux ou d'un métal des colonnes VII A, VIII, I B, IV B et V B de la classification périodique des éléments ;
   - C représente Mo et/ou W ;
   - D représente le phosphore, l'arsenic, l'antimoine, le silicium, le germanium et/ou le bore;
   - E représente un élément choisi parmi le vanadium éventuellement en combinaison avec au moins un métal des colonnes VA, VIIA, VIII ou le chrome ;
   - f = a + αb avec α dépendant de la charge de l'ion B, soit égal à 2,3 ou 4 ;
   - c varie entre 5 et 20-e inclus ;
   - d varie entre 1 et 5 inclus ;
   - e varie entre 1 et 9 inclus.

2. Procédé selon la revendication précédente, caractérisé en ce que l'on effectue la réaction en présence d'un catalyseur dont la phase active est obtenue à partir d'un hétéropolyacide de formule (I) dans laquelle D est le phosphore, E le vanadium, c est compris entre 1 et 3 inclus, d vaut 1 et la somme de c+e est égale à 12.

3. Procédé selon l'une des revendications précédentes, caractérisé en ce que l'on effectue la réaction en présence d'un catalyseur dont la phase active comprend en outre un support.

4. Procédé selon la revendication précédente, caractérisé en ce que le support est choisi parmi les dioxydes de titane, silicium, zirconium, cérium, étain, l'alumine, la silice-alumine, ou leurs mélanges.

5. Procédé selon l'une quelconque des revendications précédentes, caractérisé en ce que l'on effectue la réaction avec un mélange gazeux comprenant 0,1 à 99,9 % en mole d'alcane, et plus particulièrement entre 0,1 et 3 % ou entre 10 et 99 % en mole d'alcane.

6. Procédé selon l'une quelconque de revendications précédentes, caractérisé en ce que l'on utilise comme source d'oxygène de l'air, de l'oxygène ou du protoxyde d'azote.

7. Procédé selon la revendication précédente, caractérisé en ce que l'on effectue la réaction avec un mélange gazeux comprenant 0,1 à 99,9 % en mole d'oxygène, et plus particulièrement comprenant entre 1 et 90 % ou entre 97 et 99,9 % en mole d'oxygène.

8. Procédé selon l'une quelconque des revendications précédentes, caractérisé en ce que l'on utilise un mélange gazeux présentant un rapport molaire alcane/oxygène inférieur à 20 et plus particulièrement compris entre 0,01 et 0,2 ou entre 0,6 et 18.

9. Procédé selon l'une quelconque des revendications précédentes, caractérisé en ce que l'on effectue la réaction en présence d'un diluant choisi parmi l'eau, ou les gaz inertes, ou des gaz recyclés de la réaction, seuls ou en mélange.

10. Procédé selon la revendication précédente, caractérisé en ce que l'on utilise un mélange gazeux comprenant de l'eau en tant que diluant.

11. Procédé selon la revendication précédente, caractérisé en ce que l'on utilise un mélange gazeux comprenant 0 à 70 % en mole d'eau, et de préférence comprenant 0 à 20 % en mole d'eau.

12. Procédé selon l'une des revendications 9 à 11, caractérisé en ce que l'on utilise un mélange gazeux comprenant un gaz inerte choisi parmi les gaz rares ou l'azote, en tant que diluant.

13. Procédé selon la revendication précédente, caractérisé en ce que l'on utilise un mélange gazeux comprenant 0 à 70 % en mole de gaz inerte et de préférence comprenant 0 à 20 % en mole dudit gaz.

14. Composition obtenue à partir de dioxyde de titane en tant que support et d'un hétéropolyacide de formule (I) : $[A_aB_b]_f[C_cD_dE_eO_x]^{f-}$ dans laquelle :
    - A représente au moins un cation monovalent choisi parmi l'hydrogène, un métal alcalin, ou l'ion ammonium ;
    - B représente $VO^{2+}$, $VO^{3+}$, un ion d'un métal alcalino-terreux ou d'un métal des colonnes VII A, VIII, I B, IV B et V B de la classification périodique des éléments ;
    - C représente Mo et/ou W ;
    - D représente le phosphore, l'arsenic, l'antimoine, le silicium, le germanium et/ou le bore ;
    - E représente un élément choisi parmi le vanadium éventuellement en combinaison avec au moins un métal des colonnes VA, VIIA, VIII ou le chrome ;
    - $f = a + \alpha b$ avec $\alpha$ dépendant de la charge de l'ion B, soit égal à 2,3 ou 4 ;
    - c varie entre 5 et 20-e inclus ;
    - d varie entre 1 et 5 inclus ;
    - e varie entre 1 et 9 inclus.

15. Composition selon la revendication précédente, caractérisée en ce que l'hétéropolyacide correspond à la formule (I) dans laquelle D est le phosphore, E le vanadium, c est compris entre 1 et 3 inclus, d vaut 1 et la somme de c+e est égale à 12.

16. Composition selon l'une des revendications 14 ou 15, caractérisée en ce que le dioxyde de titane est sous forme anatase, rutile, brookite, bronze ou leurs mélanges.

17. Composition selon l'une quelconque des revendications 14 à 16, caractérisée en ce que le rapport atomique (C + E) / Ti est compris entre 0,1 et 30 %, et de préférence compris entre 5 et 20 %.

18. Catalyseur caractérisé en ce qu'il comprend en tant que phase active la composition selon l'une quelconque des revendications 14 à 17.

19. Catalyseur selon la revendication précédente, caractérisé en ce qu'il comprend un inerte.

20. Catalyseur selon l'une quelconque des revendications 18 ou 19, caractérisé en ce que la composition est enrobée sur un inerte et en ce que la quantité de composition est comprise entre 0,1 à 30 % en poids, par rapport au poids total de catalyseur, et de préférence, de 2 à 20 % en poids.

21. Catalyseur selon l'une quelconque des revendications 18 ou 19, caractérisé en ce que la composition est dispersée dans un inerte et en ce que la quantité de composition est comprise entre 1 à 90 % en poids, par rapport au poids total du catalyseur.

Office européen
des brevets

**RAPPORT DE RECHERCHE EUROPEENNE**

Numero de la demande

**EP 95 40 1038**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| E | EP-A-0 627 401 (RHONE-POULENC CHIMIE) <br> * page 2, ligne 50 - page 3, ligne 12 * <br> * page 3, ligne 48 - page 4, ligne 6 * <br> * page 5, ligne 7 - ligne 12 * <br> * page 6, ligne 11 - ligne 53 * <br> * revendications 1-20 * <br> --- | 1-21 | C07C53/08 <br> C07C51/215 <br> B01J27/195 |
| Y | EP-A-0 518 548 (THE STANDARD OIL CO.) <br> * revendications 1-8 * <br> --- | 1 | |
| Y | US-A-4 250 346 (YOUNG ET AL.) <br> * colonne 5, ligne 1 - ligne 26 * <br> * revendications 1-16 * <br> --- | 1 | |
| A | EP-A-0 294 845 (UNION CARBIDE CO.) <br> * revendications 1,2 * <br> --- | 1 | |
| A | US-A-2 625 519 (HARTIG) <br> * colonne 1, ligne 23 - ligne 28 * <br> * colonne 9, ligne 37 - ligne 45 * <br> * revendication 1 * <br> ----- | 1 | DOMAINES TECHNIQUES RECHERCHES (Int.Cl.6) <br><br> C07C <br> B01J |

Le présent rapport a été établi pour toutes les revendications

| Lieu de la recherche | Date d'achèvement de la recherche | Examinateur |
|---|---|---|
| LA HAYE | 23 Août 1995 | Klag, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.....................................................................
& : membre de la même famille, document correspondant

EPO FORM 1503 03.82 (P04C02)